(19) Europäisches Patentamt
European Patent Office
Office européen des brevets

(11) **EP 3 683 219 A1**

(12) **EUROPEAN PATENT APPLICATION**


(43) Date of publication:
**22.07.2020 Bulletin 2020/30**

(51) Int Cl.:
***C07D 471/04*** $^{(2006.01)}$ ***A61P 25/28*** $^{(2006.01)}$
***A61K 31/519*** $^{(2006.01)}$

(21) Application number: **19151900.8**

(22) Date of filing: **15.01.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Julius-Maximilians-Universität Würzburg**
**97070 Würzburg (DE)**

(72) Inventors:
- **DECKER, Michael**
  **97074 Würzburg (DE)**
- **HOFFMANN, Matthias**
  **97228 Rottendorf (DE)**

(74) Representative: **Dr. Gassner & Partner mbB**
**Wetterkreuz 3**
**91058 Erlangen (DE)**


(54) **COMPOUND INHIBITING BUTYRYLCHOLINESTERASE**


(57) The invention concerns a compound according to formula

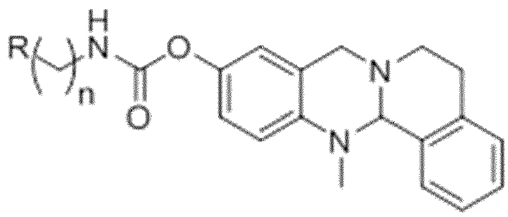

,

wherein 3 < n < 11, wherein R comprises or consists of a heterocycle comprising one tertiary amino group providing the binding of R to the rest of the molecule and at least three carbon atoms, wherein all carbon atoms of the heterocycle are unsubstituted.


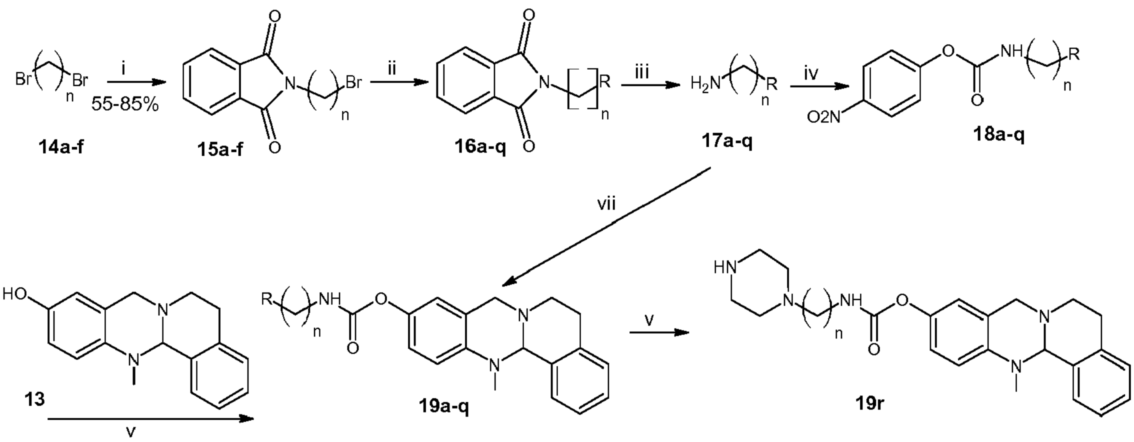


14a-f 15a-f 16a-q 17a-q 18a-q 13 19a-q 19r

**Fig. 2**

EP 3 683 219 A1

## Description

The invention concerns a compound inhibiting butyrylcholinesterase (BChE), the compound for use in the treatment of a disease, a use of that compound and a method for synthesizing that compound. The BChE can be human butyrylcholinesterase (hBChE).

The most established theory for development of Alzheimer's disease (AD) is the cholinergic hypothesis. Briefly, patients in AD suffer from an extensive loss of cholinergic neurons and cholinergic activity caused by reduced activity of choline acetylase. A rise of acetylcholine level by inhibiting its enzymatic hydrolysis by acetylcholinesterase (AChE) proved to slow down the progression of AD and had positive effect on patients' cognition and overall mental abilities in numerous clinical studies, both animal and human. Modern drugs for the treatment of AD target the two cholinesterases AChE and BChE. Among the two reversible and selective AChE inhibitors donepezil and galantamine, there is also the carbamate based drug molecule rivastigmine, targeting both enzymes in a pseudo irreversible manner. Clinical trials showed that the approved drug molecules described above lack pharmacological action and effectivity, especially in later stages of AD, whereas they slow down the progression of dementia in early stages.

It has been found that BChE activity rises in response to reduced AChE activity in later stage of AD and that BChE takes over the hydrolytic function of AChE. Therefore, inhibition of AChE is not sufficient to enhance acetylcholine (Ach) levels in this stage. BChE inhibitors could prove to be effective in the treatment of scopolamine induced amnesia and in an AD mouse model in vivo indicating their value for innovative treatments of dementia both from Alzheimer and other types. Significant progress on new cholinesterase inhibitors targeting both enzymes together has been reported in the last years, but inhibitors often lack selectivity and long term binding towards BChE.

From the doctoral thesis of Edgar Sawatzky "Design and Synthesis of Selective Butyrylcholinesterase (BChE) Inhibitors for the Development of Radiotracers to Investigate the Role of BChE in Alzheimer's Disease" selective carbamate based BChE inhibitors having a tetrahydroquinazoline carrier scaffold are known. Compounds dual-targeting the Catalytic Active Site (CAS) and the Peripheral Anionic Site (PAS) of BChE such as

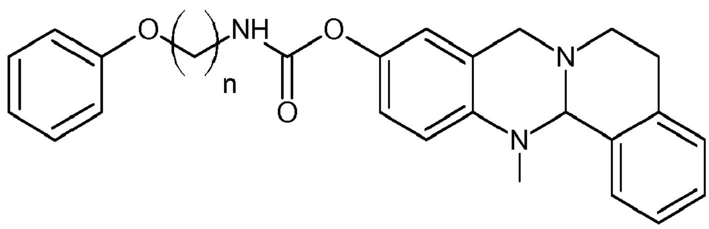

with n = 3, 5 and 7 were found to be potent and selective BChE inhibitors that lack long term binding. The postulated compound

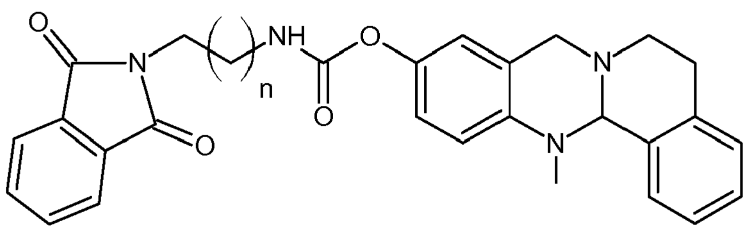

could not be synthesized.

The problem to be solved by the present invention is to provide a compound being an alternative potent and selective BChE inhibitor, the compound for use in the treatment of a disease, a use of that compound and a method for synthesizing that compound.

The problem is solved by the features of claims 1, 10, 11 and 12. Embodiments are subject-matter of claims 2 to 9 and 13 to 15.

According to the invention a compound according to formula

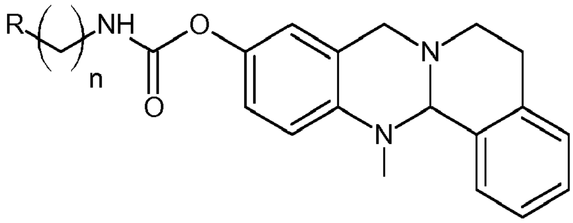

is provided, wherein 3 < n < 11, wherein R comprises or consists of a heterocycle comprising one tertiary amino group providing the binding of R to the rest of the molecule and at least three carbon atoms, wherein all carbon atoms of the heterocycle are unsubstituted. Contrary to the above-mentioned postulated compound, synthesis of the compound according to the invention could be performed in good yield without any problems. The inventors found that the compound is a potent BChE inhibitor showing pro-cognitive and antineuroinflammatory properties in vivo in an animal model of AD. In vitro experiments showed that the properties are dependent on the length of the linker formed by the n methylenegroups.

Inhibition of BChE activity was shown to be selecitve vis-à-vis AChE. Furthermore, the inhibition was shown to be pseudo-irreversble and particularly effective when n is in the range of 6 to 10, in particular when n is 6, 7 or 8 and in particular when n is 6. The inhibition is pseudo-irreversible because the carbamate moiety of the inhibitor is transferred onto the serine hydroxyl group in the catalytic center of the BChE. As a covalent bond is formed during the inhibition, the inhibitor is bound relatively fixed to the enzyme. However, due to chemical instability, this inhibition is not completely irreversible. Enzyme activity is recovered over time by carbamate hydrolysis. Duration of action of the compound on the enzyme can be controled by chemical modification and design of the carbamate moiety. Especially a linker of 6 carbon atoms between carbamate group and a basic hexacylic amine has been shown to promote the long term binding to the enzyme.

The whole heterocycle may be unsubstituted. This simplifies synthesis of the compound and enhances its enzyme inhibiting activity.

In an embodiment the heterocycle is a 5-, 6-, 7- or 8-membered ring.

R can be a fused bicyclic compound comprising the heterocycle and one further cyclic compound. The further cyclic compound may be an aromatic cyclic compound. It can be a carbocyclic compound or a heterocyclic compound. The further cyclic compound may be unsubstituted. In an embodiment the further cyclic compound is benzene.

R can be any of the following moieties:

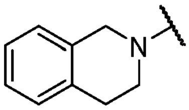, 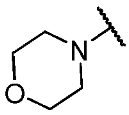, 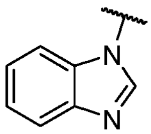, 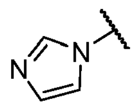, 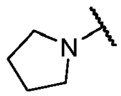, 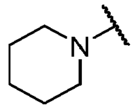, 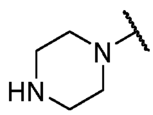

and 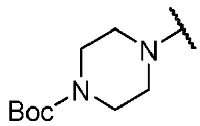,

wherein Boc is a tert-butyloxycarbonyl group. In a very effective compound according to the invention R is

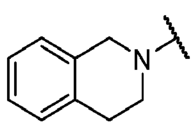 or 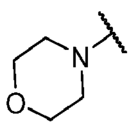.

It is particularly specific vis-à-vis inhibition of AChE activity and effective as inhibitor of BChE activity, in particular hBChE activity when n is 6, 7 or 8. In the compound most effective in vivo R was

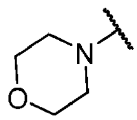

and n was 6.

The invention further concerns a compound according to the invention for use in the treatment of Alzheimer's Disease.

A further aspect of the invention concerns the use of the compound according to the invention for inhibition of butyrylcholinesterase (BChE) *in vitro.*

Another aspect of the invention concerns a method for synthesizing a compound according to the invention comprising the following steps:

a) Reacting a primary dihaloalkane comprising 4 to 10 methylene groups with a sodium or potassium salt of phthalimide to give the corresponding haloalkylated phthalimide,

b) reacting the haloalkylated phthalimide with an amine and H-R or activating the haloalkylated phthalimide in a Finkelstein reaction and then reacting the resulting iodoalkylated or fluoroalkylated phthalimide with H-R to yield an R-alkylated phthalimide,

c) subjecting the R-alkylated phthalimide to hydrazinolysis or hydrolysis to yield alkylated amines of R,

d) activating the alkylated amines of R into a carbamate by use of 4-nitrophenyl chloroformate and

e) reacting the carbamate with the tetrahydroisoquinazoline derivative according to formula

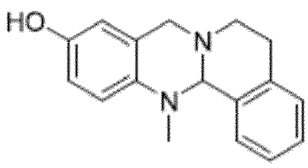

to yield the compound.

Steps a) and c) form a Gabriel-synthesis. The introduction of the residue R in step b) can be achieved in two different ways: In the first approach, the amine H-R may be stirred together with the haloalkylated phthalimide and triethyl amine in dry Dimethylformamide (DMF) to yield the R-alkyl phthalimide. In the second approach, a two-step, one pot reaction may be used by activating the haloalkylated phthalimide in a Finkelstein reaction first, and then adding the respective amine H-R to alkylate it. In some cases the second approach may result in an improved yield vis-à-vis the first approach.

Step e) may be performed by addition of an alkali hydride, in particular sodium hydride, and the tetrahydroisoquinazoline derivative to the carbamate.

In an embodiment the salt of the phthalimide is the potassium salt. The primary dihaloalkane can be a primary dibromoalkane. In this case the haloalkylated phthalimide resulting from step a) is bromoalkyl phthalimide.

R may be

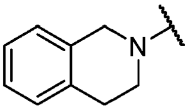 , 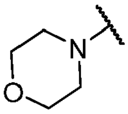 , 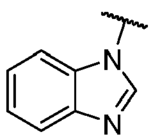 , 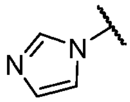 , 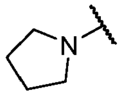 , 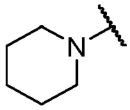 , 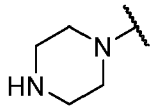 or

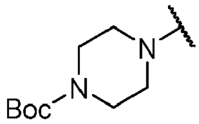 ,

wherein Boc is a tert-butyloxycarbonyl group.

The hydrazinolysis may be performed by heating the R-alkylated phthalimide in hydrazine and ethanol. The hydrolysis may be an acidic or an alkaline hydrolysis.

The reaction mixture resulting from step d) which reaction mixture comprises the carbamate may be directly subjected to step e). Directly means that the carbamate is not purified from the reaction mixture before performing step e). Step e) may be performed by addition of an alkali hydride, in particular sodium hydride, and the tetrahydroisoquinazoline derivative to the reaction mixture.

When R is

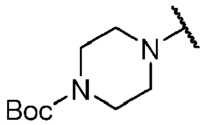

step e) may be followed by a deprotection under acidic conditions resulting in compound

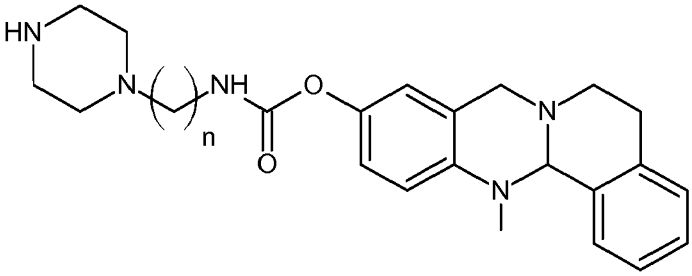

without cleaving the main pharmacophoric carbamate and the cyclic aminal structure.

Embodiments of the invention:

| | |
|---|---|
| Fig. 1 | shows a reaction scheme for the synthesis of a compound required for the synthesis of the compound according to the invention. |
| Fig. 2 | shows a reaction scheme for the synthesis of the compound according to the invention. |
| Fig. 3 | shows a diagram of the results of determination of $IC_{50}$ values of compounds according to the invention having different length of the linker. |
| Fig. 4 | shows a diagram of the results of determination of inhibition of BChE activity over time depending on the concentration of a compound according to the invention. |
| Fig. 5 | shows a diagram of the results of determination of regeneration of BChE activity over time of different compounds according to the invention. |
| Fig. 6 | shows a protocol of the experimental treatment and testing of mice. |
| Figs. 7a-h | show the effect of the compounds on $A\beta_{25\text{-}35}$-induced spontaneous alternation deficits in mice. |
| Figs. 8a-d | show the effect of the compounds on $A\beta_{25\text{-}35}$-induced passive avoidance impairments in mice. |

Synthesis of the target compounds can be divided into two parts followed by the coupling of the two building blocks in the last step. The synthesis of the tetrahydroisoquinazoline scaffold is illustrated in Fig. 1 and known from Darras et al., ACS Med. Chem. Lett. 2012, 3, 914-919 and Sawatzky, E. et al., Tetrahedron Lett. 2014, 55, 2973-2976. Briefly, 3-anthralinic acid **10** was reacted with triphosgen, followed by methylation to give anhydride **11.** Benzyl protection of the phenolic alcohol group and subsequent ring fusion with dihydroisoquinoline followed by debenzylation under hydrogenation conditions gave dihydroquinazolinone **12.** Reduction with $LiAlH_4$ gave tetrahydroisoquinazoline **13.**

Referring to Fig. 1 reagents and reaction conditions were as follows: (i) 1. $CO(CCl_3)_2$, THF, 70°C, 3-5 h; 2. Methyl iodide, DIPEA, DMAc, 40°C, 24h; (ii) 1. Benzyl bromide, $K_2CO_3$, DMF, 40°C, 4h; 2. Dihydroisoquinoline, DMF,

120°C, 18 h; 3. $H_2$, Pd/C, MeOH, 50°C, 3 h; (iii) $LiAlH_4$, THF, reflux, 3 h.

To investigate how a peripheral binding site of BChE can be addressed with carbamate based inhibitors, different alkylene linkers and residues were introduced into the carbamate part of the inhibitors by using Gabriel synthesis as illustrated in Fig. 2 in combination with Table 1. Therefor, different dibromo alkanes **14a-f** of length from 2 to 10 methylene groups were reacted in each case with potassium phthalimide in DMF to give the alkylated phthalimides **15a-f** in high yields. For the introduction of the amine residues two different strategies were applied:

In a first approach, the respective amine, such as morpholine or 1,2,3,4-tetrahydrosioquinolin, was stirred together with the respective bromoalkyl phthalimide **15a-f** and triethyl amine in dry DMF to yield the alkylated amine derivatives **16a-l**.

In a second approach, a two-step, one pot reaction was used by activating the bromoalkane **15d** in a Finkelstein reaction first, and then adding the respective amine to alkylate it, which gave access to improved yields of **16m-q.**

Subsequent hydrazinolysis in ethanol under reflux conditions gave respective amines **17a-q** in high yields. Amines were then activated into respective carbamates **18a-q** by using 4-nitrophenyl chloroformate, which carbamates were then transferred onto the tetrahydroisoquinazoline scaffold **13** to yield the respective carbamate based BChE inhibitors **19a-q.**

Deprotection of Boc-protected piperazine **19q** under acidic conditions yielded another prospective inhibitor **19r** without cleaving the main pharmacophoric carbamate and the cyclic aminal structure.

Referring to Fig. 2 reagents and reaction conditions were as follows: (i) Potassium phthalimide, DMF, 24 h, r.t. (ii) a) R-H, $NEt_3$, DMF, 24 h, 100°C or b) NaI, $NEt_3$, THF, o.n. reflux, 60-91% (iii) $N_2H_4{\cdot}H_2O$, ethanol, 18 h, reflux, 90-100% (iv) 4-Nitrophenylchloroformate, $NEt_3$, DCM, 1-6 h, r.t., 20-86%; (v) NaH, DCM (dry), 1-48 h, r.t., 30-80%; (vi) 1.25 M HCl in methanol, ethanol (dry), 4.5 h, 0°C → r.t., 96%; (vii) 1. 4-Nitrophenylchloroformate, $NEt_3$, DCM, 1-6 h, r.t., no purification, 2. NaH, **13,** DCM (dry), 1-48 h, r.t., 40-60%. For substitution pattern of n and R see table 1. Pathway vii is an alternative pathway to separate pathways iv and v. In pathway vii the reaction mixture resulting from pathway iv is directly - i. e. without purification of carbamates **18a-q** - used for reacting the carbamate contained in the reaction mixture with the tetrahydroisoquinazoline derivative **13.**

Table 1. Overview on synthesized compounds according to Fig. 2

| | | n | R | | | n | R |
|---|---|---|---|---|---|---|---|
| **14-15** | **a** | 2 | - | **16-19** | **g** | 2 | 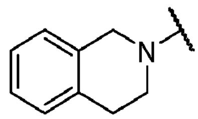 |
| **14-15** | **b** | 3 | - | **16-19** | **h** | 3 | 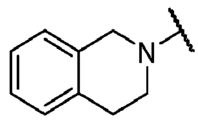 |
| **14-15** | **c** | 4 | - | **16-19** | **i** | 4 | 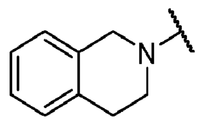 |
| **14-15** | **d** | 6 | - | **16-19** | **j** | 6 | 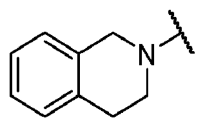 |
| **14-15** | **e** | 8 | - | **16-19** | **k** | 8 | 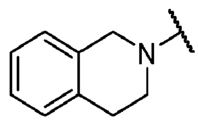 |
| **14-15** | **f** | 10 | - | **16-19** | **l** | 10 | 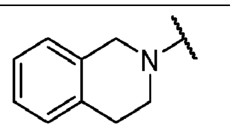 |

(continued)

| | | n | R | | | n | R |
|---|---|---|---|---|---|---|---|
| **16-19** | **a** | 2 | 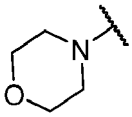 | **16-19** | **m** | 6 | 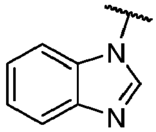 |
| **16-19** | **b** | 3 | 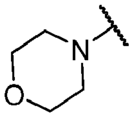 | **16-19** | **n** | 6 | 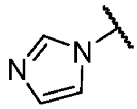 |
| **16-19** | **c** | 4 | 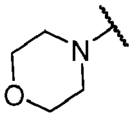 | **16-19** | **o** | 6 | 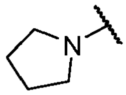 |
| **16-19** | **d** | 6 | 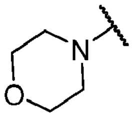 | **16-19** | **P** | 6 | 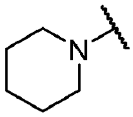 |
| **16-19** | **e** | 8 | 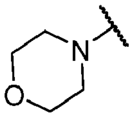 | **16-19** | **q** | 6 | 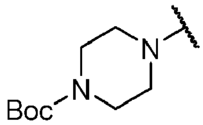 |
| **16-19** | **f** | 10 | 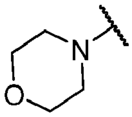 | **19** | **r** | 6 | 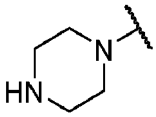 |

For evaluation of enzyme inhibition, Ellman's colorimetric assay was conducted as described in Ellman, G. L., Arch. Biochem. Biophys 1958, 74, 443-450 and Ellman, G. L. et al., Biochem. Pharmacol. 1961, 7, 88-95. Therefor, 5,5'-dithiobis-(2-nitrobenzoic acid) (DTNB) solution, respective Cholinesterase (ChE) and inhibitor were incubated together and enzyme activity was measured after 20 min. The long incubation time was needed to allow the carbamate transfer between ChE and inhibitor.

The carbamate residue was altered systematically by changing residues in the terminal position of carbamate moiety and different flexible alkylene linkers were introduced. Obtained enzyme inhibition constants were reviewed with respect to benchmark heptyl carbamate **7** described in Darras, F. H. et al., ACS Med. Chem. Lett. 2012, 3, 914-919. As compound **7** shows low nanomolar inhibition of human BChE (*h*BChE) and approximately 1000-fold higher inhibition of human AChE (*h*AChE), it serves as a perfect lead compound for further optimizations because of its selectivity, high potency and plasma stability. Nevertheless, it must be noted that $IC_{50}$ values only serve as a first indicator of inhibitor affinity and selectivity. Due to the chemical reaction of carbamate transfer the enzyme inhibition is strongly time dependent. Kinetic investigations indicated that 20 min is usually sufficient to allow carbamate transfer.

Table 2. Inhibition of *h*BChE and *h*AChE by carbamate based inhibitors **7, 9, 19a-r** and binding of these inhibitors to *h*BChE and *h*AChE

| 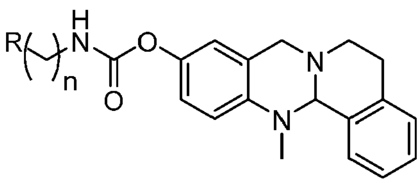 | R | n | $IC_{50}$ hBChE [nM] ($pIC_{50}$ ± SEM) | $IC_{50}$/ inhibition of hAChE ($pIC_{50}$ ± SEM) | Selectivity index ($IC_{50}$ hAChE/$IC_{50}$ hBChE) |
|---|---|---|---|---|---|
| 7 | $CH_3$ | 6 | 6.4* (8.19 ± 0.02) | 3.8 μM (5.42 ± 0.05) * | 594 |

(continued)

| 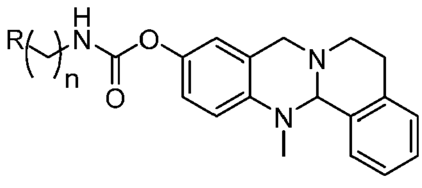 | | | IC$_{50}$ hBChE [nM] (pIC$_{50}$ ± SEM) | IC$_{50}$/ inhibition of hAChE (pIC$_{50}$ ± SEM) | Selectivity index (IC$_{50}$ hAChE/IC$_{50}$ hBChE) |
|---|---|---|---|---|---|
| | **R** | **n** | | | |
| 9 | F | 7 | 5.2* (8.28 ± 0.02) | 3.6 μM (5.44 ± 0.05) * | 692 |
| 19a | 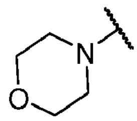 | 2 | 7772 (5.11 ± 0.04) | 17%[a] | >10 |
| 19b | 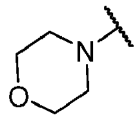 | 3 | 937.8 (6.03 ± 0.02) | 13%[a] | >100 |
| 19c | 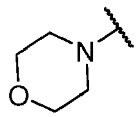 | 4 | 50.6 (7.30 ± 0.02) | 13%[a] | >1000 |
| 19d | 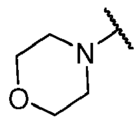 | 6 | 49.3 (7.31 ± 0.02) | 45%[a] | >1000 |
| 19e | 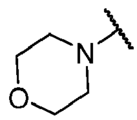 | 8 | 19.8 (7.07 ± 0.06) | 22%[a] | >1000 |
| 19f | 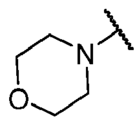 | 10 | 11.5 (7.94 ± 0.07) | 25%[a] | >1000 |
| 19g | 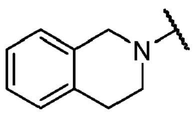 | 2 | 1720 (5.76 ± 0.05) | 10%[b] | >10 |
| 19h | 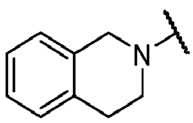 | 3 | 1160 (5.94 ± 0.05) | 12%[b] | >10 |
| 19i | 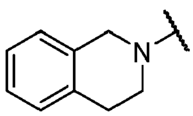 | 4 | 191.7 (6.72 ± 0.06) | 16%[b] | >100 |
| 19j | 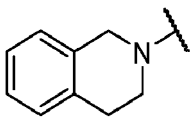 | 6 | 26.7 (7.57 ± 0.05) | 9%[b] | >1000 |
| 19k | 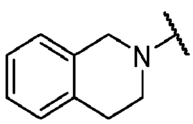 | 8 | 32.6 (7.49 ± 0.03) | 11%[b] | >1000 |

(continued)

| 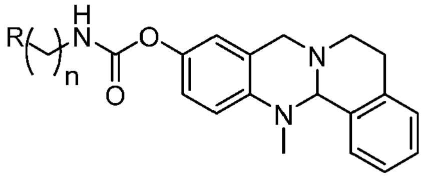 | | | $IC_{50}$ hBChE [nM] ($pIC_{50}$ ± SEM) | $IC_{50}$/ inhibition of hAChE ($pIC_{50}$ ± SEM) | Selectivity index ($IC_{50}$ hAChE/$IC_{50}$ hBChE) |
|---|---|---|---|---|---|
| | **R** | **n** | | | |
| 19l | 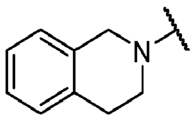 | 10 | 41.8 (7.38 ±0.04) | 15%[b] | >1000 |
| 19m | 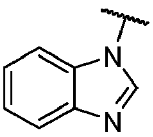 | 6 | 7.4 (8.13 ± 0.04) | 44%[c] | >1000 |
| 19n | 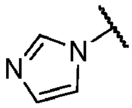 | 6 | 13.3 (7.88 ± 0.06) | 18%[c] | >1000 |
| 19o | 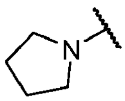 | 6 | 34.3 (6.47 ± 0.04) | 0.88 μM (6.06 ± 0.03) | 26 |
| 19p | 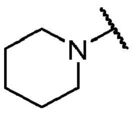 | 6 | 83.7 (7.08 ± 0.04) | 1.2 μM (5.91 ± 0.03) | 14 |
| 19q | 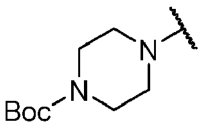 | 6 | 123.4 (6.1 ± 0.04) | 15%[c] | >100 |
| 19r | 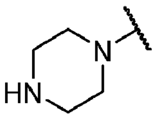 | 6 | 80.6 (7.09 ± 0.03) | 16%[c] | >100 |

[a]100 μM; [b]10 μM [c]25 μM
* According to Sawatzky, E. et al., ChemMedChem 2016, 11, 1540-1550

Table 2 shows that inhibition of *h*BChE and *h*AChE by carbamate based inhibitors **7, 9** and **19a-r** and binding of these inhibitors to *h*BChE and *h*AChE depend on linker length n and residue R.

To investigate the influence of length of carbon chain linker between carbamate group and residue possibly targeting a second binding site located near the catalytical active serin in the center of *h*BChE, a set of compounds **19a-f** having a morpholine derivative residue and a further set of compounds **19g-l** having a tetrahydroisoquinoline derivative residues were investigated. The compounds in each of the two sets differ from each other by the number n of carbon atoms in the carbon chain linker between carbamate group and residue. Fig. 3 shows the $IC_{50}$ values obtained with *h*BChE and the two compound sets having morpholine **19a-f** (symbol "**+**") and tetrahydroisoquinoline derivatives **19g-l** (symbol "**x**") as residues. Fig. 3 shows a strong dependency of the $IC_{50}$ values on the number n of carbon atoms in the carbon chain linker. From the $IC_{50}$ values it can be concluded, that short linker length influence enzyme inhibition in a negative manner. Elongation of the linker to 4 methylene groups as seen for compounds **19c** and **19i** lead to a significant increase of inhibitory potency showing two-digit nanomolar $IC_{50}$ values comparable to known inhibitors **7** and **9.** Further elongation of linker length improves the activity of the morpholine compounds slightly, while for the tetrahydroisoquinoline compounds a linker length of 6 methylene groups (**19j**) is optimal and the affinity decreases with a longer linker. Nevertheless, $IC_{50}$ of heptyl carbamate **7** cannot be achieved with polar residues such as morpholine, piperidine, pyrrolidine

and piperazine, but approximately 10-fold lower activity of the compounds **19d, 19o** and **19p** still proves the finding of new very potent and even more selective BChE inhibitors. Most $IC_{50}$ values of the investigated compounds on *h*AChE could not be determined due to their poor water solubility and low affinity.

From the binding data presented here, it can be concluded that *h*BChE tolerates a huge variety of residues, when the linker is longer than 4, better 6 methylene groups. Both very polar groups like morpholine and sterically demanding groups like tetrahydroisoquinoline and benzimidazole do not alter the inhibition of *h*BChE in a significant manner. These binding data do not indicate the presence of a second binding site in *h*BChE that can be addressed with the inhibitors.

As carbamate based inhibitors (C-X) show a pseudo irreversible manner of binding, the $IC_{50}$ values must be seen critically because two chemical reactions determine them: Carbamate (C) transfer onto the enzyme (E) and carbamate hydrolysis. So, the $K_c$ values, $k_3$ and $k_4$ rate constants according to below formula were investigated for some representative compounds (c.f. Fig. 3 and Fig. 4).

$$\underbrace{E + \text{C-X} \underset{k_2}{\overset{k_1}{\rightleftharpoons}} (\text{EC-X})}_{K_c} \xrightarrow[\text{-X}]{k_3} \text{E-C} \xrightarrow[\text{-C}']{k_4} E$$

The mechanism of enzyme inhibition can be divided into three steps: In a pre-equilibrium, the carbamate based inhibitor C-X is bound to the enzyme E and a complex (EC-X) is formed reversible and can be characterized with $K_c$. Then the carbamate C is transferred onto the enzyme E and binds covalently to form E-C with the rate constant $k_3$ while carrier scaffold X is released. Then E-C is hydrolyzed and enzyme activity is recovered and the amine C' is released with the rate constant $k_4$, also called decarbamoylation rate.

For the determination of $K_c$ and $k_3$ a similar experimental setup was used compared to the evaluation of $IC_{50}$ values, whereas enzyme activity was measured at various time points after addition of inhibitor.

Thereby, inhibition with the carbamate-based inhibitors showed a first order kinetic with the apparent first order rate constant $k_{obs}$, where A is the enzyme activity at time t, $A_0$ the enzyme activity at t = 0 and $A_\infty$ the enzyme activity at t = ∞.

$$A = A_0 \cdot e^{-k_{obs} \cdot t} + A_\infty \qquad (I)$$

Plotting the enzyme activity (in %) against the incubation times, lead to nonlinear time-dependent inhibition curves for the respective inhibitor concentrations whereby $k_{obs}$ can be determined with an exponential fit correspondent to equation (I). Fig. 4 shows the time-dependent inhibition of relative enzyme activity (rel. enz. act.) by different concentrations of compound **19j** on *h*BChE.

Furthermore, a hyperbolic curve is obtained, plotting the rate constant $k_{obs}$ against the inhibitor concentration [I], which is described by equation (II), with the substrate concentration [S] and the Michaelis-Menten constant $K_M$.

$$k_{obs} = \frac{k_3 \cdot [I]}{K_C \cdot \left(1 + \frac{[S]}{K_M}\right) + [I]} \qquad (II)$$

This equation (II) can be rearranged to the reciprocal of $k_{obs}$, to get a linear plot:

$$\frac{1}{k_{obs}} = \frac{K_C \cdot \left(1 + \frac{[S]}{K_M}\right)}{k_3} \cdot \frac{1}{[I]} + \frac{1}{k_3} \qquad (III)$$

Since the substrate butyrylthiocholine iodide is not added directly to the enzyme and the inhibitor, but after the

incubation of enzyme with inhibitor, [S] can be set to zero, leading to Michaelis-Menten like equation IV.

$$\frac{1}{k_{obs}} = \frac{K_C}{k_3} \cdot \frac{1}{[I]} + \frac{1}{k_3} \qquad \text{(IV)}$$

Relating to the linear equation (IV), $K_C$ can be established from the slope and carbamoylation rate constant $k_3$ from the y-intercept.

As a chemical reaction is taking place, $K_c$ must be seen as a 'real' measure for quantification of enzyme inhibitor interactions. Due to the high costs in measurements on *h*BChE only some representative compounds were chosen to evaluate also in kinetic studies based on the results from $IC_{50}$ measurements (table 3).

Table 3. Kinetic values for carbamoylation on *h*BChE for selected representative compounds

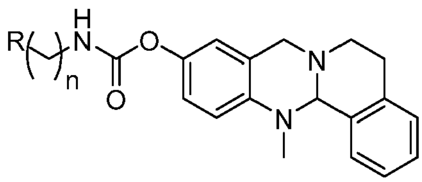

| | **R** | **n** | $K_c$ [nM] *h*BChE | $k_3$ [$min^{-1}$] *h*BChE |
|---|---|---|---|---|
| 7 | $CH_3$ | 6 | 28.5 ± 17.2 * | 0.66 ± 0.32 * |
| 9 | F | 7 | 24.3 ± 19.3 * | 0.78 ± 0.48 * |
| 19d | 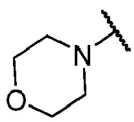 | 6 | 202 ± 55 | 0.16 ± 0.02 |
| 19g | 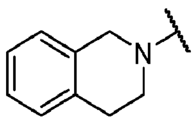 | 2 | >200000 | >20 |
| 19j | 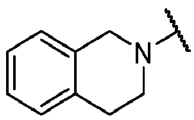 | 6 | 3.6 ± 2.9 | 0.18 ± 0.02 |
| 19l | 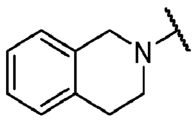 | 10 | 5.68± 2.19 | 0.25 ± 0.04 |
| 19m | 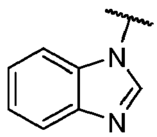 | 6 | 7.7 ± 4.6 | 0.26 ± 0.09 |
| 19n | 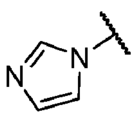 | 6 | 13.1 ± 14.4 | 0.31 ± 0.15 |
| 19q | 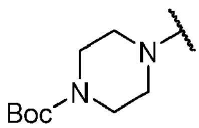 | 6 | 1549 ± 827 | 0.70 ± 0.31 |

(continued)

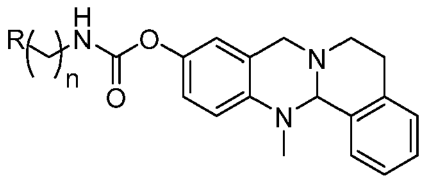

| | R | n | $K_c$ [nM] *h*BChE | $k_3$ [min$^{-1}$] *h*BChE |
|---|---|---|---|---|
| 19r | 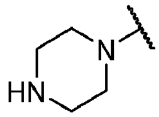 | 6 | 121 ± 116 | 0.32 ± 0.13 |

* According to Sawatzky, E. et al., ChemMedChem 2016, 11, 1540-1550

From the carbamoylation rate constants $k_3$ it can be concluded, that linker and residue do not have a big influence on the carbamate transfer from the carrier scaffold to the enzyme. No additional effect of a peripheral binding site on the binding of inhibitor could be found. The only exception is tetrahydroisoquinoline compound **19g** probably forcing the inhibitor into a different binding mode because of its geometry with a very bulky residue close to the carrier scaffold. This confirms the results of the $IC_{50}$ measurements.

From this set of compounds, compound **19j** must be noted with special interest as its $K_c$ value is even smaller than the $IC_{50}$ value which is rarely the case. This might indicate a peripheral binding as $K_c$ characterizes the equilibrium of reversible binding (Fig. 4), whereas $IC_{50}$ also includes pseudo-irreversible binding (carbamoylation). Nevertheless, it must be noted that $IC_{50}$ gives an inhibitor concentration, whereas $K_c$ is a dissociation constant and therefore cannot be interpreted as concentration. Therefore, it is remarkable to reach very low $K_c$ values in the one-digit nanomolar range, showing that inhibitors **19j, l, m** and **n** show a very high affinity to *h*BChE and outreach lead compound **7** in this regard.

To further investigate the presence and nature of a peripheral binding site, also the hydrolysis of carbamate and the resulting release of the inhibitor from the enzyme was investigated by measuring the time dependent recovery of enzyme activity. To measure the decarbamoylation rate $k_4$, dilution experiments were performed with *h*BChE following the procedure described in Sawatzky, E. et al., ChemMedChem 2016, 11, 1540-1550 and in Bartolucci, C. et al., Biochem. J. 2012, 444, 269-277. A high concentration of enzyme and inhibitor where incubated for 1 h. The concentration of inhibitor was chosen, such that > 85% of *h*BChE was inhibited through carbamate transfer. Then the mixture was diluted with buffer 1:1000 to a concentration at which the enzyme is not further carbamoylated. Due to the hydrolysis and release of the carbamate from the catalytic active serine enzyme activity was recovered slowly. This regeneration of the enzyme was monitored at different time points after the dilution. Plotting the time dependent enzyme activity against time showed a first order exponential curve, with the associated equation (V).

$$A = \left(1 - e^{k_4 \cdot t}\right) \cdot \left(1 - A_0\right) + A_0 \qquad (V)$$

Table 4. Kinetic values for decarbamoylation from *h*BChE of selected compounds

| | R | n | $k_4$ [h$^{-1}$] | $t_{1/2}$ [E-C] [h] |
|---|---|---|---|---|
| 7 | $CH_3$ | 6 | 0.62 ± 0.04 * | 1.12 ± 0.07 * |
| 9 | F | 7 | 0.81 ±0.04 * | 0.86 ± 0.04 * |
| 19c | 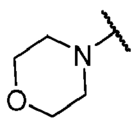 | 4 | 0.48 ± 0.06 | 1.44 ± 0.16 |
| 19d | 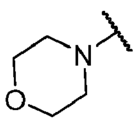 | 6 | 0.042 ± 0.004 | 16.50 ± 1.44 |

(continued)

| | R | n | $k_4$ [$h^{-1}$] | $t_{1/2}$ [E-C] [h] |
|---|---|---|---|---|
| 19e | 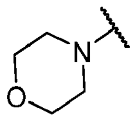 | 8 | 1.58 ± 0.15 | 0.44 ± 0.04 |
| 19i | 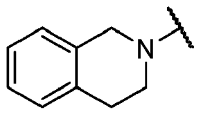 | 4 | 0.028 ± 0.001 | 24.76 ± 0.85 |
| 19j | 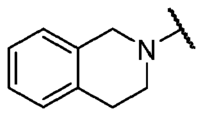 | 6 | 0.025 ± 0.001 | 27.73 ± 1.07 |
| 19k | 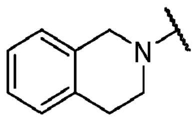 | 8 | 0.83 ± 0.06 | 0.84 ± 0.06 |
| 19m | 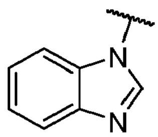 | 6 | 0.45 ± 0.04 | 1.55 ± 0.13 |
| 19n | 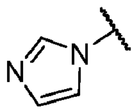 | 6 | 0.82 ± 0.03 | 0.85 ± 0.03 |
| 19o | 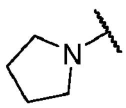 | 6 | 0.98 ± 0.04 | 0.71 ± 0.03 |
| 19p | 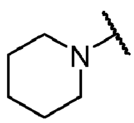 | 6 | 0.129 ± 0.006 | 5.37 ± 0.24 |
| 19r | 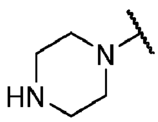 | 6 | 1.44 ± 0.07 | 0.48 ± 0.02 |

* According to Sawatzky, E. et al., ChemMedChem 2016, 11, 1540-1550

In case of the morpholine compounds a significant difference is observed comparing compounds **19c** and **19d** with 4 and 6 methylene groups in the linker, respectively. While the kinetic of hydrolysis of compound **19c** is comparable to the heptyl carbamate reference compound **7,** morpholine compound **19d** shows a significantly reduced speed of enzyme regeneration with a half-life time $t_{1/2}$ = 16.3 h compared to 1.4h for compound **19c** and 1.1 h for reference compound 7. For a linker length of n = 8 in compound **19e** the half-life time is decreased significantly again, so that a linker of 6 methylene groups was considered best. This novel finding indicates the presence of a second binding site because the enzyme is recovered considerably slower and since due to the alkylene linker attached to the carbamate moiety formed with the serine, no difference in chemical reactivity of the carbamate is expected. The time dependent enzyme regeneration from carbamoylated state [E-C] from compounds **19d**(*), **19c**(+), **7** (x) is shown in Fig. 5

In contrast to the morpholine compounds the tetrahydroisoquinoline compounds did not show this behavior in such a pronounced manner. Nevertheless, two tested inhibitors showed a very long duration of inhibition. Compound **19i** and **19j** inhibit the enzyme for a long duration, while a linker length of n = 8 (**19k**) decreases the duration of inhibition as it was observed and described above. This finding could be explained by the fact, that the residue is bigger and can

target a peripheral binding site also with shorter linkers.

The finding of a slower decarbamoylation of carbamate inhibitors from *h*BChE could be explained both by a specific binding of the residues to a second binding region and/or unspecific blocking of water molecules from the serine in the CAS slowing down the chemical reaction of hydrolysis. Consequently, to distinguish between those two explanations more inhibitors with a linker length of n = 6 methylene groups were synthesized, including very different residues with basic and non-basic, aliphatic and aromatic, polar and non-polar properties.

In contrast to our expectation the aromatic compounds with benzimidazole **19m** and imidazole residues **19n** did not show improved duration of enzyme inhibition, despite their very promising binding and affinity data. This leads to the conclusion that a specific interaction between the transferred carbamate residue is generally possible, but aromatic residues do not address the PAS specifically. In case of an unspecific steric inhibition of carbamate hydrolysis it can be expected to observe a high stability of the carbamoylated enzyme inhibitor complex (E-C) (c.f. Fig. 4) when bulky residues like benzimidazole are introduced into the inhibitor.

In analogy to AChE inhibitors basic residues proved to be more promising for a binding to a PAS. Despite their comparably slightly deteriorated binding properties, most compounds bearing a basic amine function and a linker length of six methylene groups showed a decelerated hydrolysis behavior compared to the reference compound **7.**

This leads to the conclusion that both *h*AChE and *h*BChE possess a second binding site, which can be addressed with carbamate inhibitors and affects their duration of binding dramatically.

Generally, also size and shape of residue seems to play a role for deceleration of carbamate hydrolysis, whereas hydrolysis of benzimidazole carbamate **19m** ($t_{1/2}$ = 1.55 $\pm$ 0.13 h) is slower than imidazole carbamate **19n** ($t_{1/2}$ = 0.85 $\pm$ 0.03 h). The accelerated hydrolysis of pyrrolidine compound **19o** ($t_{1/2}$ = 0.71 $\pm$ 0.03 h) in comparison to the bigger piperidine carbamate **19p** ($t_{1/2}$ = 5.37 $\pm$ 0.24 h) is in agreement with this result.

Another explanation for this finding could be found in the conformation of the residues. The best compounds from the half-life aspect, all share a tert-piperidine moiety (tetrahydroisoquinoline, morpholine and piperidine) and exhibit a chair conformation. In this conformation, the most potent compounds **19d, 19i** and **19j** all possess an electron rich system (oxygen atom or benzene ring, respectively) adding another affinity, which seems to further enhance carbamate stability compared to piperidine compound **19p.**

*In vivo* studies

Compounds **7, 19d, 19j** and **13** were tested for their neuroprotectant and pro-cognitive properties in an *in vivo* model of AD in mice as described before in Dolles, D. et al., J. Med. Chem. 2018, 61, pages 1646-1663, Maurice, T. et al., Brain Res. 1996, 706, pages 181-193 and Lahmy, V. et al., Neuropsychopharmacol. 2013, 38, pages 1706-1723.

AD-like cognitive dysfunctions were induced in male Swiss mice, 6 weeks old, weighing 31-36 g by intracerebroventricular (icv) injection of the oligomerized $A\beta_{25-35}$ peptide into the mouse brain on day 1.

Compounds were weighed, dissolved in pure DMSO at a concentration of 1 mg/ml and diluted into final test concentrations with saline. The final percentage of DMSO in saline was 60% for all compounds. Vehicle solution used for control groups was 60% DMSO in saline. Compounds were injected intraperitoneally (ip) from day 1 to 7 and behavioral studies were conducted between day 8 and 10. Spatial working memory was evaluated using a spontaneous alternation test in a Y-maze on day 8. The Y-maze is made of grey polyvinylchloride. Each arm is 40 cm long, 13 cm high, 3 cm wide at the bottom, 10 cm wide at the top, and converging at an equal angle. Each mouse is placed at the end of one arm and allowed to explore the maze freely for 8 min. The sequence of arm entries (including possible returns into the same arm) was checked visually and noted down. If the mouse enters all three arms on consecutive occasion, this is defined as an alternation. Therefore, the total number of arm entries minus two is also the maximum number of alternations. The percentage of alternation was calculated as (actual alternations / maximum alternations) x 100. Parameters for the evaluation of behavior are given as the percentage of alternation (memory index) and a total number of arm entries (exploration index). When an extreme behavior (Alternation percentage < 20% or > 90% or a number of arm entries < 10) was observed, animals were excluded from the calculations, which corresponded to a 4.7% attrition in this study.

Non-spatial long-term memory was analyzed using a step-through passive avoidance test, with training on day 9 and retention on day 10. The setup for the experiment consists of a two-compartment (15 x 20 x 15 cm high) polyvinylchloride box, whereas one compartment is white and illuminated with a bulb (60 W, 40 cm above the apparatus) and the other black with a cover and grid floor. The two compartments are separated by a guillotine door. On day 9, during the training session, each animal was placed in the white compartment with the door closed. After 5 s, the door was opened and the mouse was allowed to enter the dark compartment. When it had placed all its paws on the grid floor, the door was closed and a foot shock was delivered (0.3 mA) for 3 s using a scrambled shock generator (Lafayette Instruments, Lafayette, USA). The time spent to enter the dark compartment (step-through latency), and the level of sensitivity to the shock was evaluated (no sign=0, flincing reactions=1, vocalizations=2). None of the treatment affected the step-through latency or shock sensitivity in the present study (data not shown). The retention test was carried out

on day 10. 5 s after the mouse was placed in the white compartment, the door was opened and the mouse was allowed to explore the box. The time spent to enter the dark compartment (step-through latency) was measured up to 300 s. Animals showing latencies during the training and retention session lower than 10 s and shock sensitivity = 0 were considered as failing to learn the task and discarded from calculations. In this study, it corresponded to 0.6% attrition.

A protocol of the experimental treatment and testing of mice is illustrated in Fig. 6. The meaning of the abbreviations used in Fig. 6 is as follows: icv, intracerebroventricular injection; YMT, spontaneous alternation test in the Y-maze; ST-PA, step-through passive avoidance test.

Statistical analyses

All values, except passive avoidance latencies, are expressed as mean $\pm$ S.E.M. Statistical analyses were performed on the different conditions using one-way ANOVA ($F$ value), followed by the Dunnett's *post-hoc* multiple comparison test. Passive avoidance latencies do not follow a Gaussian distribution since upper cutoff times (300 s) are defined. Therefore, they were analyzed using a Kruskal-Wallis non-parametric ANOVA ($H$ value), followed by a Dunn's multiple comparison test. $p < 0.05$ was considered as statistically significant.

Results

*Repeated treatments with the compounds*

None of the treatments (ip for the compounds partially solubilized in DMSO and icv for the peptide) affected significantly the mouse body weight gain during the week of treatment showing a good tolerability. Animals gained 2.2-4.5 g during the 7 days treatment period and particularly recovered quickly from the icv injection stress after day 1.

*Spatial working memory measure using the spontaneous alternation performance in the Y-maze*

Figs. 7a-h show the effect of the compounds on $A\beta_{25-35}$-induced spontaneous alternation deficits in mice. Mice received $A\beta_{25-35}$ (9 nmol icv) or vehicle (V) solution (3 $\mu$l icv) on day 1 and compounds **7** (Figs. 7a, b), **19d** (Figs. 7c, d), **19j** (Figs. 7e, f) and **13** (Figs. 7g, h), in the 0.3-3 mg/kg ip dose range once per day (*o.d.*) from day 1 to 7. Figs. 7a, c, e, g: spontaneous alternation performance; Figs. 7b, d, f, h: number of arm entries in the Y-maze test performed at day 8. Data show mean $\pm$ SEM. ANOVA: $F_{(4,79)}$ = 2.61, p < 0.05, n = 13-18 in (a); $F_{(4,79)}$ = 1.32, p > 0.05 in (b); $F_{(4,61)}$ = 4.06, p < 0.01, n = 11-13 in (c); $F_{(4,61)}$ = 1.00, p > 0.05 in (d); $F_{(4,63)}$ = 4.30, p < 0.01, n = 11-15 in (e); $F_{(4,63)}$ = 4.50, p < 0.01 in (f); $F_{(4,63)}$ = 3.07, p < 0.05, n = 11-15 in (g); $F_{(4,63)}$ = 2.59, p < 0.05 in (h). * $p$ < 0.05, ** $p$ < 0.01 vs. (V+V)-treated group; # $p$ < 0.05, ## $p$ < 0.01, ### $p$ < 0.001 vs. ($A\beta_{25-35}$+V)-treated group; Dunnett's test.

Compound **7** attenuated the $A\beta_{25-35}$-induced spontaneous alternation deficits at 1 and 3 mg/kg ip doses (Fig. 7a). However, the increases in alternation percentage remained non-significantly different from the $A\beta_{25-35}$-treated group. Compounds **19d** and **19j** appeared very active since they were active at lower doses: 0.3 mg/kg ip for **19d** and 1 mg/kg ip for **19j** (Figs. 7c, e). Interestingly, the phenolic compound **13** lacking BChE inhibition was not active in this response (Fig. 7g). It must be noted that the $A\beta_{25-35}$ treatment failed to affect exploratory response, measured in terms of the number of arms entered during the session (Figs. 7b, d, f, h). Noteworthily, all compounds tended to slightly increase locomotor activity (Figs. 7b, d, f, h) and in a significant manner for compound **19j** (Fig. 7f).

Long-term memory assessment using the passive avoidance test

Figs. 8a-d show the effect of the compounds on $A\beta_{25-35}$-induced passive avoidance impairments in mice. Mice received $A\beta_{25-35}$ (9 nmol icv) or vehicle solution (3 $\mu$l icv) on day 1 and compounds **7** (Fig. 8a), **19d** (Fig. 8b), **19j** (Fig. 8c) and **13** (Fig. 8d), in the 0.3-3 mg/kg ip dose range o.d. from day 1 to 7. Animals were trained in the passive avoidance test on day 9, and retention (step-through latency) was analyzed on day 10. Data show median and interquartile range. Kruskal-Wallis ANOVA: $H$ = 12.3, p < 0.05, n = 16-18 in (a); $H$ = 21.1, p < 0.05, n = 11 - 15 in (b); $H$ = 13.5, p < 0.01, n = 11-14 in (c); $H$ = 11.8, p < 0.01, n = 11-12 in (d). * $p$ < 0.05, ** $p$ < 0.01, *** $p$ < 0.001 vs. (V+V)-treated group; # $p$ < 0.05, ## $p$ < 0.01 vs. ($A\beta_{25-35}$+V)-treated group; Dunn's test.

Compound **7** significantly prevented the $A\beta_{25-35}$-induced passive avoidance deficits at all dose tested (Fig. 8a). Both compounds **19d** and **19j** were effective at the 3 doses tested and particularly at the lowest 0.3 mg/kg ip dose (Figs. 8b, c).

Compound **13** was not active, with only a few animals showed improved latency at the highest dose (Fig. 8d).

In summary, as compared to the parent compound **7,** compounds **19d** and **19j** showed very promising effects on both investigated behavioral parameters. They particularly fully prevented the effects of $A\beta$ injection at a dose of 1 or 3 mg/kg ip.

the *in vivo* results prove the penetration of the blood brain barrier, despite the high lipophilicity and molecular weight of compounds.

Abbreviations:

ACh, Acetylcholine; AChE, Acetylcholinesterase; Aβ, amyloid β; AD, Alzheimer's disease; ATC, acetyl thiocholine iodide; BChE, Butyrylcholinesterase; BTC, butyryl thiocholine iodide; eq, equation; DTNB, 5,5'-dithiobis-(2-nitrobenzoic acid); icv, intracerebroventricular; ip intraperitoneal; o.d., once per day; V, vehicle, ST-PA, step-through passive avoidance test; YMT, spontaneous alternation test in the Y-maze;

## Claims

1. Compound according to formula

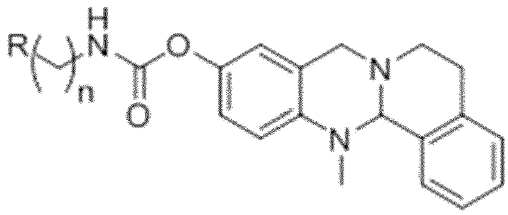

,

wherein 3 < n < 11, wherein R comprises or consists of a heterocycle comprising one tertiary amino group providing the binding of R to the rest of the molecule and at least three carbon atoms, wherein all carbon atoms of the heterocycle are unsubstituted.

2. Compound according to claim 1, wherein the total heterocycle is unsubstituted.

3. Compound according to claim 1 or 2, wherein the heterocycle is a 5-, 6-, 7- or 8-membered ring.

4. Compound according to any of the preceding claims, wherein R is a fused bicyclic compound comprising the heterocycle and one further cyclic compound.

5. Compound according to claim 4, wherein the further cyclic compound is an aromatic cyclic compound.

6. Compound according to claim 4 or 5, wherein the further cyclic compound is a carbocyclic compound or a heterocyclic compound.

7. Compound according to any of claims 4 to 6, wherein the further cyclic compound is unsubstituted.

8. Compound according to claim 7, wherein the further cyclic compound is benzene.

9. Compound according to any of the preceding claims, wherein R is

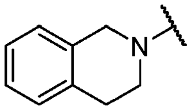 , 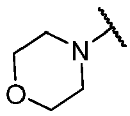 , 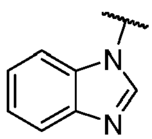 , 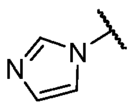 , 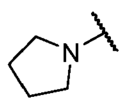 , 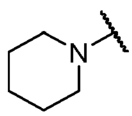 , 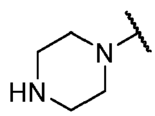 or

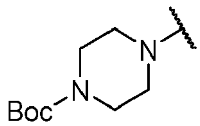 ,

wherein Boc is a tert-butyloxycarbonyl group, in particular wherein R is

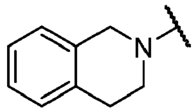 or 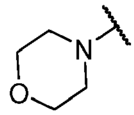,

in particular wherein R is

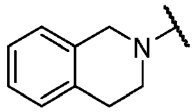 or 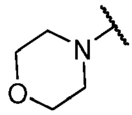,

and n is 6, 7 or 8.

**10.** Compound according to any of the preceding claims for use in the treatment of Alzheimer's Disease.

**11.** Use of a compound according to any of claims 1 to 9 for inhibition of butyrylcholinesterase (BChE) *in vitro.*

**12.** Method for synthesizing a compound according to any of claims 1 to 9 comprising the following steps:

a) Reacting a primary dihaloalkane comprising 4 to 10 methylene groups with a sodium or potassium salt of phthalimide to give the corresponding haloalkylated phthalimide,
b) reacting the haloalkylated phthalimide with an amine and H-R or activating the haloalkylated phthalimide in a Finkelstein reaction and then reacting the resulting iodoalkylated or fluoroalkylated phthalimide with H-R to yield an R-alkylated phthalimide and
c) subjecting the R-alkylated phthalimide to hydrazinolysis or hydrolysis to yield alkylated amines of R,
d) activating the alkylated amines of R into a carbamate by use of 4-nitrophenyl chloroformate and
e) reacting the carbamate with the tetrahydroisoquinazoline derivative according to formula

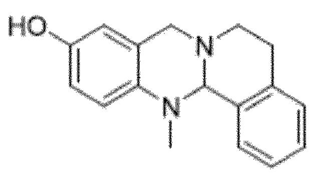

to yield the compound.

**13.** Method according to claim 12, wherein the salt of the phthalimide is the potassium salt and/or the primary dihaloalkane is a primary dibromoalkane and/or R is

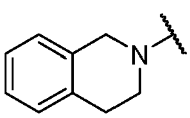, 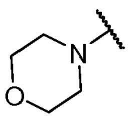, 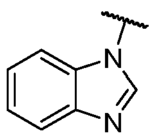, 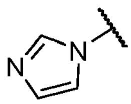, 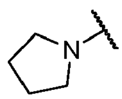, 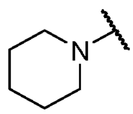, 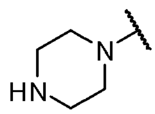 or

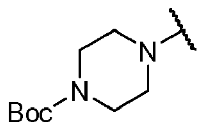,

wherein Boc is a tert-butyloxycarbonyl group, and/or the hydrazinolysis is performed by heating the R-alkylated

phthalimide in hydrazine and ethanol.

**14.** Method according to claim 12 or 13, wherein a reaction mixture resulting from step d) which reaction mixture comprises the carbamate is directly subjected to step e).

**15.** Method according to any of claims 12 to 14, wherein R is

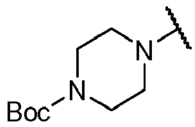

and step e) is followed by a deprotection under acidic conditions.

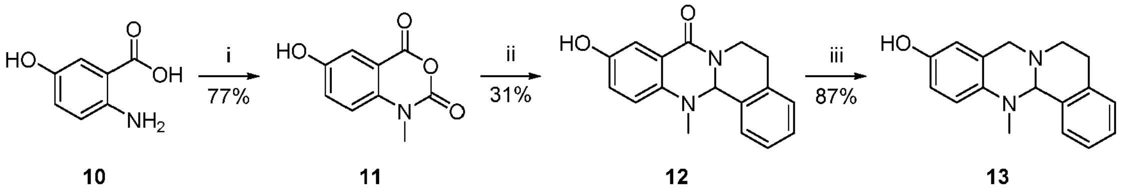


**Fig. 1**

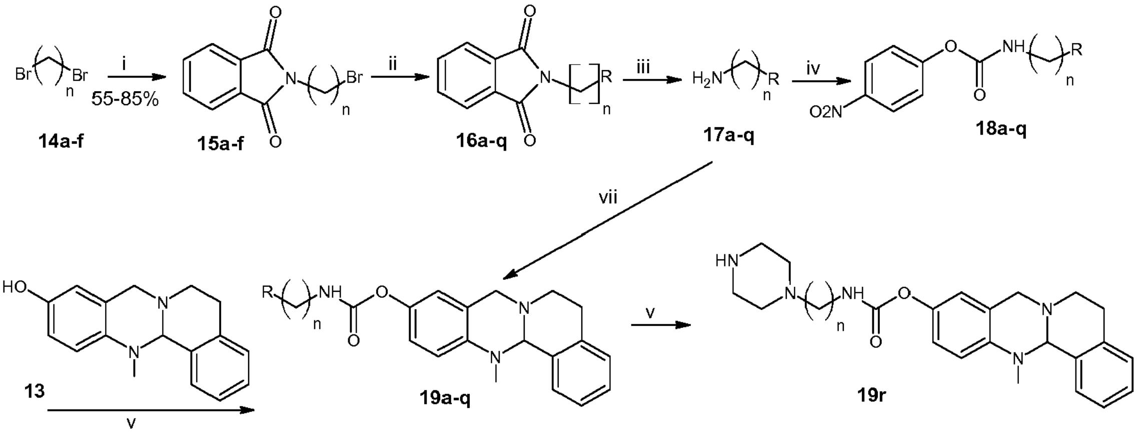


**Fig. 2**

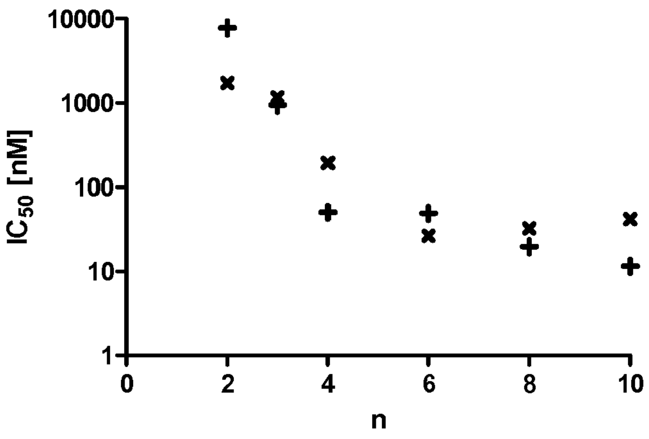


**Fig. 3**

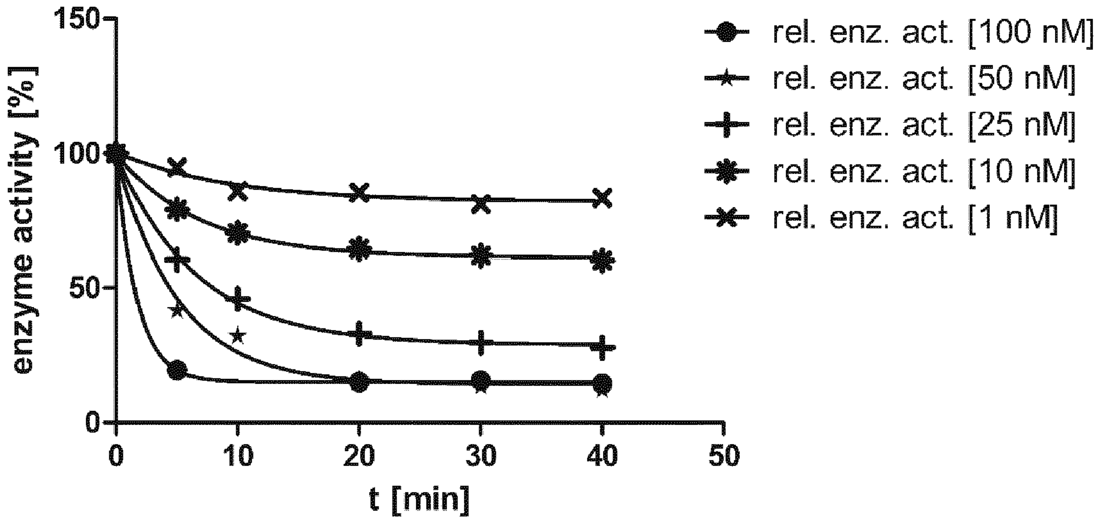
150
100
50
0
enzyme activity [%]
0
10
20
30
40
50
t [min]
rel. enz. act. [100 nM]
rel. enz. act. [50 nM]
rel. enz. act. [25 nM]
rel. enz. act. [10 nM]
rel. enz. act. [1 nM]

Fig. 4

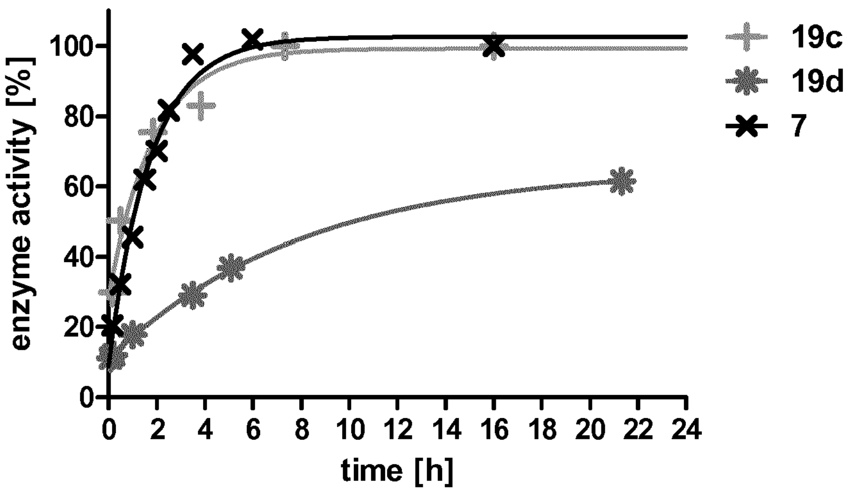
100
80
60
40
20
0
enzyme activity [%]
0 2 4 6 8 10 12 14 16 18 20 22 24
time [h]
19c
19d
7

Fig. 5

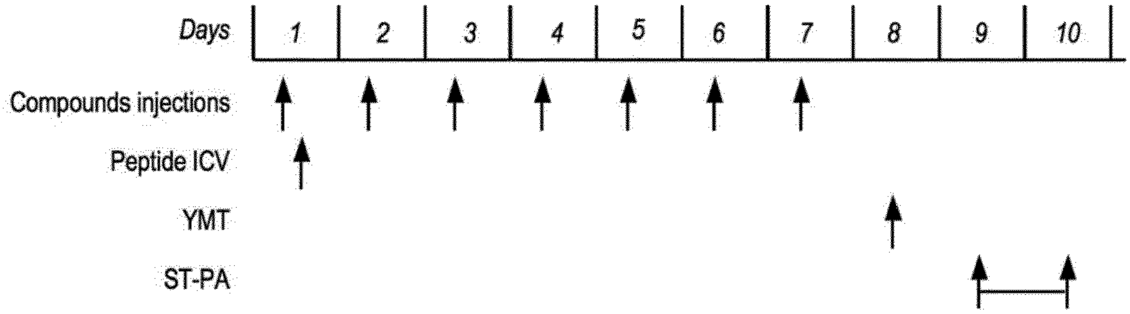
Days
1
2
3
4
5
6
7
8
9
10
Compounds injections
Peptide ICV
YMT
ST-PA

Fig. 6

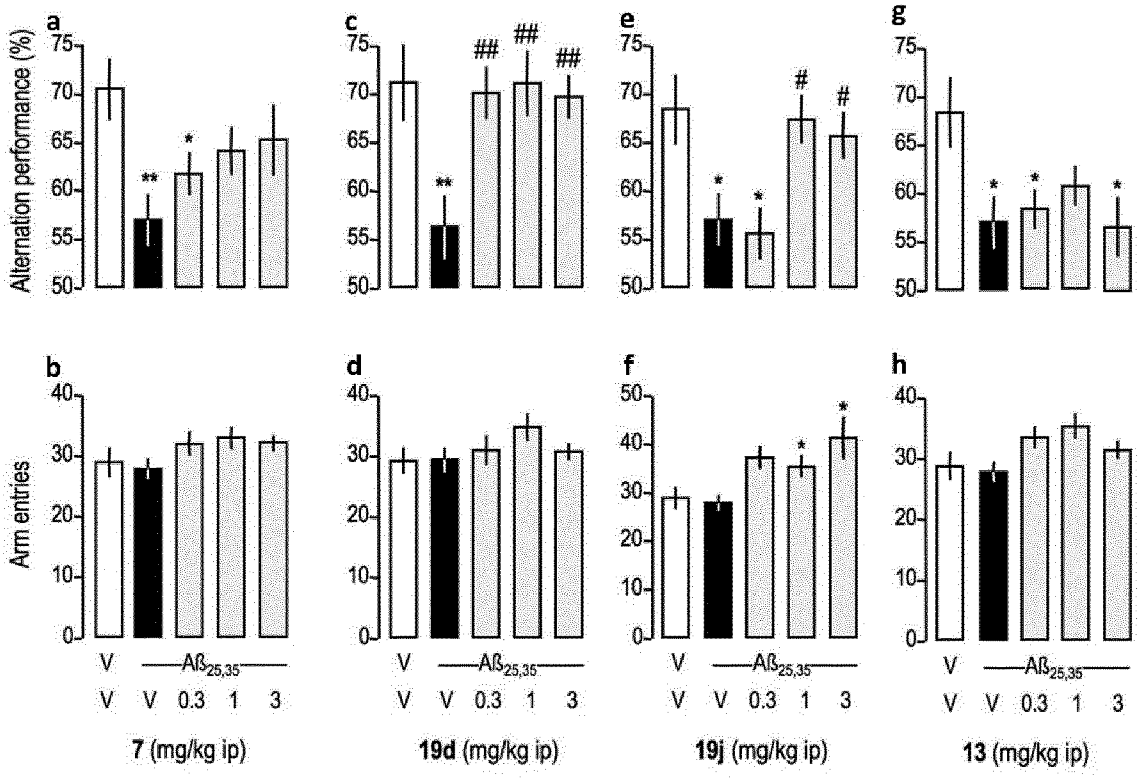
a
b
c
d
e
f
g
h
Alternation performance (%)
Arm entries
Aß25,35
V V V 0.3 1 3
7 (mg/kg ip)
19d (mg/kg ip)
19j (mg/kg ip)
13 (mg/kg ip)

Fig. 7

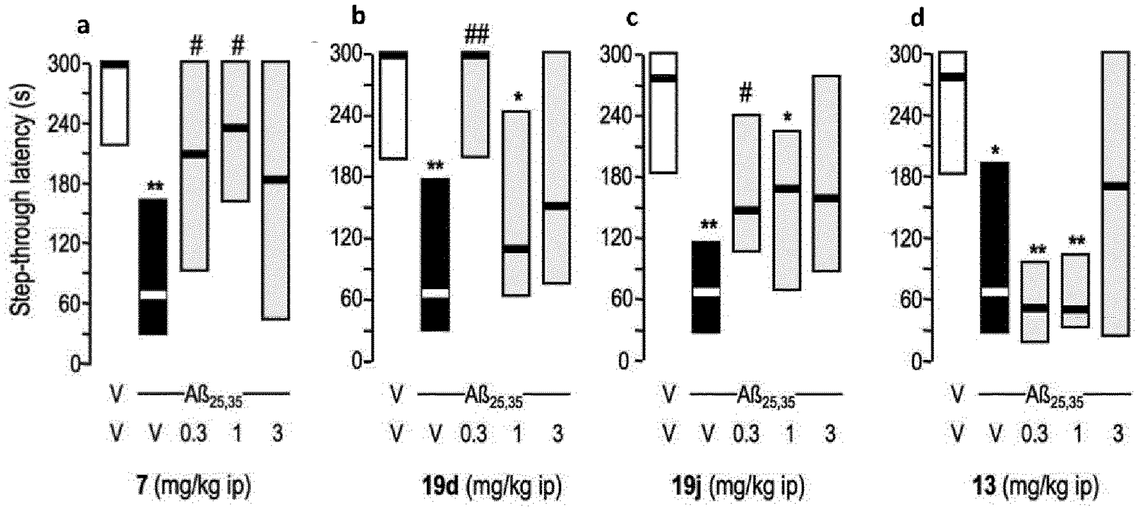
a
b
c
d
Step-through latency (s)
300
240
180
120
60
0
#
##
*
**
V
Aß25,35
V V 0.3 1 3
7 (mg/kg ip)
19d (mg/kg ip)
19j (mg/kg ip)
13 (mg/kg ip)

**Fig. 8**

Europäisches Patentamt
European Patent Office
Office européen des brevets

# EUROPEAN SEARCH REPORT

Application Number
EP 19 15 1900

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br>A | Edgar Sawatzky: "Design and Synthesis of Selective Butyrylcholinesterase (BChE) Inhibitors for the Development of Radiotracers to Investigate the Role of BChE in Alzheimer's Disease", Dissertation, University of Würzburg, 17 February 2016 (2016-02-17), pages 63-71, XP055593417, Retrieved from the Internet: URL:https://opus.bibliothek.uni-wuerzburg.de/opus4-wuerzburg/frontdoor/deliver/index/docId/14403/file/Sawatzky_Edgar_Butyrylcholinesterase.pdf [retrieved on 2019-06-03]<br>* Scheme 5.1; "Conclusion"; table 5.1; compound 5 *<br>----- | 1-3, 10-12,14<br><br>9,13,15 | INV.<br>C07D471/04<br>A61P25/28<br>A61K31/519 |

TECHNICAL FIELDS SEARCHED (IPC)

C07D
A61P
A61K

The present search report has been drawn up for all claims

| Place of search | Date of completion of the search | Examiner |
|---|---|---|
| Munich | 24 June 2019 | Johnson, Claire |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **DARRAS et al.** *ACS Med. Chem. Lett.,* 2012, vol. 3, 914-919
- **SAWATZKY, E. et al.** *Tetrahedron Lett.,* 2014, vol. 55, 2973-2976
- **ELLMAN, G. L.** *Arch. Biochem. Biophys,* 1958, vol. 74, 443-450
- **ELLMAN, G. L. et al.** *Biochem. Pharmacol.,* 1961, vol. 7, 88-95
- **DARRAS, F. H. et al.** *ACS Med. Chem. Lett.,* 2012, vol. 3, 914-919
- **SAWATZKY, E. et al.** *ChemMedChem,* 2016, vol. 11, 1540-1550
- **BARTOLUCCI, C. et al.** *Biochem. J.,* 2012, vol. 444, 269-277
- **DOLLES, D. et al.** *J. Med. Chem.,* 2018, vol. 61, 1646-1663
- **MAURICE, T. et al.** *Brain Res.,* 1996, vol. 706, 181-193
- **LAHMY, V. et al.** *Neuropsychopharmacol.,* 2013, vol. 38, 1706-1723